(19) Europäisches Patentamt
European Patent Office
Office européen des brevets

(11) Veröffentlichungsnummer: **0 081 719**
**B1**

# (12) EUROPÄISCHE PATENTSCHRIFT

(45) Veröffentlichungstag der Patentschrift: **23.04.86**

(51) Int. Cl.[4]: **C 07 D 233/91**, C 07 D 403/12, A 61 K 31/415

(21) Anmeldenummer: **82110919.6**

(22) Anmeldetag: **25.11.82**

(54) **2-Nitroimidazole.**

(30) Priorität: **30.11.81 CH 7657/81**
**28.09.82 CH 5701/82**

(43) Veröffentlichungstag der Anmeldung: **22.06.83 Patentblatt 83/25**

(45) Bekanntmachung des Hinweises auf die Patenterteilung: **23.04.86 Patentblatt 86/17**

(84) Benannte Vertragsstaaten: **AT BE CH DE FR IT LI NL SE**

(56) Entgegenhaltungen:
**DE - A - 2 229 223**
**LU - A - 76 007**

(73) Patentinhaber: **F. HOFFMANN-LA ROCHE & CO. Aktiengesellschaft, CH-4002 Basel (CH)**

(72) Erfinder: **Hofheinz, Werner, Dr., Talmattweg 7, CH-4103 Bottmingen (CH)**

(74) Vertreter: **Lederer, Franz, Dr. et al, Patentanwälte Dr. Franz Lederer Dipl.-Ing. Reiner F. Meyer-Roxlau Lucile-Grahn-Strasse 22, D-8000 München 80 (DE)**

EP 0 081 719 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft neue 2-Nitroimidazole der allgemeinen Formel I

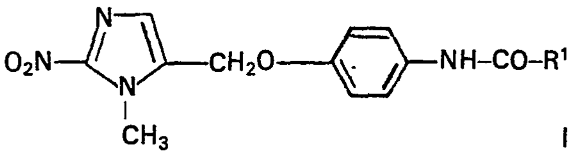

I

worin $R^1$ 1-Methyl-2-pyrrolidinyl oder einen Rest $-CH_2-NR^2R^3$ bedeutet, in dem $R^2$ und $R^3$ jeweils $C_{1-4}$-Alkyl darstellen oder $-NR^2R^3$ der Rest eines fünf- oder sechsgliedrigen gesättigten heterocyclischen Ringsystems mit ggf. einem weiteren Stickstoff- oder Sauerstoffatom im Ring ist, und ihre physiologisch verträglichen Säureadditionssalze, deren Herstellung, pharmazeutische Präparate auf der Basis der neuen Verbindungen sowie diese Verbindungen als Heilmittel.

Die $C_{1-4}$-Alkylreste können gerad- oder verzweigtkettig sein; bevorzugt sind Methyl und Ethyl. Beispiele für fünf- oder sechsgliedrige, ggf. ein weiteres Stickstoff- oder Sauerstoffatom im Ring enthaltende heterocyclische Ringsysteme sind Pyrrolidin, Piperidin, Piperazin und Morpholin.

Das erfindungsgemässe Verfahren ist dadurch gekennzeichnet, dass man

(a) eine Verbindung der Formel II

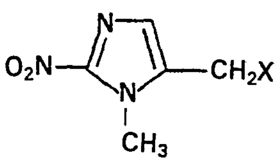

II

in der X Hydroxy, Chlor, Brom oder Jod darstellt, mit einer Verbindung der Formel III

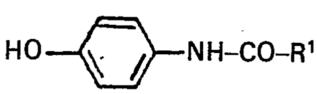

III

in der $R^1$ wie oben definiert ist, umsetzt oder

(b) 2-Halo-α- (1-methyl-2-nitroimidazol-5-yl)-p-acetanisidid mit einer Verbindung $HNR^2R^3$, in der $R^2$ und $R^3$ wie oben definiert sind, umsetzt oder

(c) α- (1-Methyl-2-nitroimidazol-5-yl) -p-anisidin mit einer Verbindung $R^1$-COOH oder einem reaktionsfähigen Derivat davon umsetzt und gewünschtenfalls eine erhaltene Verbindung I in ein physiologisch verträgliches Säureadditionssalz überführt.

Bei den Verfahren (a), (b) und (c) handelt es sich um Kondensationsreaktionen, die im Rahmen üblicher, dem Fachmann geläufiger Bedingungen durchgeführt werden können, d.h. in einem der bekannten inerten Lösungsmittel, bei Temperaturen zwischen Raumtemperatur und der Rückflusstemperatur des Reaktionsgemisches, gewünschtenfalls in Gegenwart eines Kondensationsmittels. Als reaktionsfähige Derivate von Verbindungen $R^1$-COOH (Variante c) kommen beispielsweise Säurehalogenide oder -anhydride in Betracht.

Die erfindungsgemässen neuen 2-Nitroimidazole weisen protozoocide, insbesondere trypanosomicide Aktivität auf. Gegenüber bekannten Verbindungen mit annerkannt guter trypanosomicider Wirkung, wie z.B. Pentamidin oder Suramin, zeichnen sie sich durch ausgezeichnete Liquorgängigkeit aus, während sie sich gegenüber bereits bekannten 2-Nitroimidazolen gleicher Wirkungsrichtung, wie z.B. Benznidazol oder Misonidazol, durch wesentlich stärkere Wirkung gegenüber afrikanischen Trypanosomen (z.B. Trypanosoma rhodesiense = Erreger der Schlafkrankheit) auszeichnen (vgl. Tabelle 1).

Ein bevorzugtes Anwendungsgebiet der erfindungsgemässen Verbindungen ist die Veterinärmedizin und zwar dort, wo Trypanosomenerkrankungen eine bedeutende Rolle spielen. Hervorzuheben sind die Nagana (Erreger: T. congolense, T. vivax, T. brucei) die Surra (Erreger: T. evansi) und die Dourine (T. equiperdum).

Tabelle 1:

| Verbindung | $ED_{50}$ [mg/kg], T. rhodesiense p.o. |
|---|---|
| Misonidazol | 400 |
| Benznidazol | 400 |
| 2-(Dimethylamino) -α- (1-methyl-2-nitroimidazol-5-yl) -p-acetanisidid | 3 |
| 2-(Diethylamino) -α- (1-methyl-2-nitroimidazol-5-yl) -p-acetanisidid | 17 |
| 2-(Dibutylamino) -α- (1-methyl-2-nitroimidazol-5-yl) -p-acetanisidid | 90 |
| α- (1-Methyl-2-nitroimidazol-5-yl)-1-pyrrolidin-p-acetanisidid | 2,7 |
| α- (1-Methyl-2-nitroimidazol-5-yl)-1-piperidin-1- acetanisidid | 55 |
| α- (1-Methyl-2-nitroimidazol-5-yl)-4-morpholin-p-acetanisidid | 30 |
| (2S) 1-Methyl-α- (1-methyl-2-nitroimidazol-5-yl) -pyrrolidin-carboxy-p-anisidid | 18 |

Die erfindungsgemässen Verbindungen können daher als Arzneimittel und zwar zur Prophylaxe und Therapie von durch Protozoen, insbesondere Trypanosomen, hervorgerufenen Krankheiten, wie der Schlafkrankheit, Verwendung finden. Sie können in Form von festen oder flüssigen pharmazeutischen Präparaten in Mischung mit für die orale oder parenterale Applikation geeigneten organischen oder anorganischen inerten

Trägermaterialien sowie den üblichen Hilfsstoffen appliziert werden. Als Dosierungsrichtlinie kann beim Menschen eine Menge von 1–10 mg aktiver Substanz/kg Körpergewicht pro Tag während einer Behandlungszeit von 1–10 Tagen gelten. Die Dosierungseinheit liegt zweckmässigerweise bei 50–1000 mg. Eine analoge Dosierung gilt auch für die Veterinärmedizin.

Die erfindungsgemässen Verbindungen I können in den pharmazeutischen Präparaten in Form von Additionssalzen mit bekannten physiologisch verträglichen Säuren, z.B. Salzsäure, Schwefelsäure, Phosphorsäure, Essigsäure, Citronensäure, Weinsäure, vorliegen. Die Herstellung dieser Salze kann durch Umsetzung der Verbindungen I mit der gewünschten Säure in an sich bekannter Weise erfolgen.

Die Herstellung der erfindungsgemässen Verbindungen wird durch die beiliegenden Beispiele erläutert.

Beispiel 1

78,5 g 1-Methyl-2-nitroimidazol-5-methanol wurden in 1,5 l wasserfreiem Tetrahydrofuran (THF) suspendiert. Nach Zusatz von 102 g p-Chloracetylaminophenol und 144 g Triphenylphosphan wurde auf 10 °C gekühlt und unter starkem Rühren innert 20 Minuten eine Lösung von 111 g Azodicarbonsäure-diisopropylester in 750 ml wasserfreiem THF zugesetzt, wobei die Temperatur mittels Eisbad auf 10–12 °C gehalten wurde. Das Gemisch wurde über Nacht im Kühlschrank aufbewahrt und der kristalline Niederschlag abgenutscht. Nach Umkristallisation aus 2 l Acetonitril wurden 90,9 g (56,6%) reines 2-Chlor-α- (1-methyl-2-nitroimidazol-5-yl)-p-acetanisidid, Smp. 192–194 °C (Zers.), erhalten.

Zu einer Suspension von 20 g des erhaltenen Anisidids in 500 ml Ethanol wurden 32 ml einer 3,68 molaren Lösung von Dimethylamin in Ethanol gegeben. Es wurde 5 Stunden unter Rückfluss gekocht, unter vermindertem Druck zu Trockne eingeengt und mit 1 l Methylenchlorid und 400 ml Wasser aufgenommen. Das Gemisch wurde mit Natriumbicarbonat auf pH 7 eingestellt. Dann wurde die organische Phase abgetrennt, mit wenig Wasser gewaschen und eingedampft. Durch Kristallisation des Rückstandes aus 200 ml Isopropanol wurden 16,6 g (82%) 2-(Dimethylamino) -α- (1-methyl-2-nitroimidazol-5-yl) -p-acetanisidid, Smp. 128–129 °C, erhalten.

15 g 2-(Dimethylamino) -α- (1-methyl-2-nitroimidazol-5-yl) -p-acetanisidid wurden in 300 ml Ethanol heiss gelöst. Unter Rühren tropfte man 12 ml 4,1 N ethanolische Salzsäure zu, kühlte im Eisbad ab und gab 250 ml Diethylether zu. Nach Stehenlassen über Nacht im Kühlschrank saugte man das ausgefällte Hydrochlorid ab, wusch mit Diethylether und trocknete im Vakuum. Man erhielt 16,4 g 2-(Dimethylamino) -α- (1-methyl-2-nitroimidazol-5-yl) -p-acetanisidid-hydrochlorid, Smp. 193–194 °C (Zersetzung).

Beispiel 2

7 g 2-Chlor-α- (1-methyl- 2-nitroimidazol-5-yl ) -p-acetanisidid und 3,8 g Pyrrolidin wurden 5 Stunden in 200 ml Ethanol unter Rückfluss erhitzt. Das Gemisch wurde auf etwa 50 ml eingeengt und abgekühlt. Umkristallisation des Niederschlages aus Acetonitril lieferte 5,9 g (76%) α- (1-Methyl-2-nitroimidazol-5-yl) -1-pyrrolidin-p-acetanisidid, Smp. 141–143 °C.

15 g α- (1-Methyl-2-nitroimidazol-5-yl) -1-pyrrolidin-p-acetanisidid wurden in 350 ml Ethanol heiss gelöst. Unter Rühren tropfte man 11 ml 4,1 N ethanolische Salzsäure zu, kühlte im Eisbad ab und gab 250 ml Diethylether zu. Nach Stehenlassen über Nacht im Kühlschrank nutschte man ab, wusch mit Diethylether und trocknete im Vakuum. Man erhielt 15,4 g α- (1-Methyl-2-nitroimidazol-5-yl) -1-pyrrolidin-p-acetanisidid-hydrochlorid, Smp. 209–210 °C (Zersetzung).

Beispiel 3

In zu Beispiel 2 analoger Weise wurden aus 4,6 g Piperidin 6,1 g (75%) α-(1-Methyl-2-nitroimidazol-5-yl) -1-piperidin-p-acetanisidid, Smp. 162–163 °C, erhalten.

Beispiel 4

In zu Beispiel 2 analoger Weise wurden aus 3,95 g Diethylamin 5 g (64%) 2-(Diethylamino) -α- (1-methyl-2- nitroimidazol-5-yl) -p-acetanisidid, Smp. 106–108 °C (aus Toluol), erhalten.

Beispiel 5

In zu Beispiel 2 analoger Weise wurden aus 3,24 g 2-Chlor-α- (1-methyl-2-nitroimidazol-5-yl) -p-acetanisidid und 2,18 g Morpholin 3,3 g (83%) α- (1-Methyl- 2-nitroimidazol-5-yl) -4-morpholin-p-acetanisidid, Smp. 154–156 °C (aus Methanol), erhalten.

Beispiel 6

Eine Suspension von 30 g 1-Methyl-2-nitroimidazol-5-methanol, 22 g 4-Aminophenol und 55 g Triphenylphosphan in 600 ml wasserfreiem THF wurde unter starkem Rühren innert 20 Minuten tropfenweise mit einer Lösung von 42,5 g Azodicarbonsäure-diisopropylester in 300 ml wasserfreiem THF versetzt. Durch externe Kühlung wurde das Gemisch auf einer Temperatur von 20–24 °C gehalten. Nach 2 Stunden wurde die erhaltene klare Lösung unter vermindertem Druck eingedampft und der Rückstand mit einem Gemisch aus 125 ml Methylenchlorid und 125 ml Essigester kurz aufgekocht. Es wurden 24,4 g (52%) kristallines α- (1-Methyl-2-nitroimidazol-5-yl) -p-anisidin, Smp. 168–170 °C, erhalten.

Eine Lösung von 4,14 g N-Methyl-L-prolin und 6,2 g des erhaltenen Anisidins in 250 ml Acetonitril wurde mit 12,5 ml Triethylamin und anschliessend mit 8,5 g 2-Fluor-1-methylpyridinium-tosylat versetzt. Es wurde 1 Stunde unter Rückfluss erhitzt, das Lösungsmittel unter vermindertem Druck abgezogen und der Rückstand zwischen 200 ml Essigester und 200 ml Wasser verteilt. Die wässrige Phase wurde mit NaOH auf

pH 10 eingestellt und dreimal mit je 100 ml Essigester extrahiert. Die vereinigten Extrakte wurden eingedampft. Der Rückstand wurde mit Aceton/Toluol (1:1, v/v) über 280 g Kieselgel filtriert. Nach einem Vorlauf von 350 ml wurden aus den nächsten 2000 ml des Eluats 8,1 g (2S)-1-Methyl-α(1-methyl-2-nitroimidazol-5-yl) -pyrrolidin-carboxy-p-anisidid in Form eines öligen Rohprodukts erhalten. Umkristallisation aus Ether lieferte 2,5 g (27%) reines Produkt, Smp. 106–107 °C, $[\alpha]_D^{25}$ = −66,5° (c=1%, Methanol).

Beispiel 7

Zu einer Lösung von 22 g 1-Methyl-2-nitro-imidazol- 5-methanol in 300 ml THF und 150 ml Dimethylformamid (DMF) wurde bei einer Temperatur von −60 °C innert 15 Minuten eine Lithium-diisopropylamin-Lösung (hergestellt aus 65 ml einer 2,2 molaren Lösung von Butyllithium in Hexan und 14,5 g Diisopropylamin in 150 ml THF) zugetropft. Das Gemisch wurde 30 Minuten bei −60 °C gerührt und mit 28 g p-Toluol-sulfonylchlorid in 150 ml THF versetzt. Nach Entfernung der Kühlung, Erwärmung auf Zimmertemperatur und Zusatz von 300 ml Eiswasser wurde dreimal mit jeweils 300 ml Essigester extrahiert. Die vereinigten Extrakte wurden mit 150 ml gesättigter Kochsalzlösung gewaschen und unter vermindertem Druck eingedampft. Der Rückstand wurde an 800 g Kieselgel mit Essigester/Dichlormethan (1:3, v/v) gereinigt. Nach einem Vorlauf von 1,5 l wurden die nächsten 1,5 l gesammelt und eingedampft. Umkristallisation aus 70 ml Toluol lieferte 12 g (50%) 5-(Chlormethyl) -1-methyl-2-nitroimidazol, Smp. 100–101 °C.

Zu einer Lösung von 1,26 g 4′-Hydroxy-1-pyrrolidin-acetanilid in 10 ml DMF wurden bei −10 °C 2,9 ml einer 1,98 molaren Lösung von Natrium-tert.-amylat in Toluol gegeben. Anschliessend wurde 1 g 5-(Chlormethyl) -1-methyl-2-nitroimidazol zugesetzt und während 3 Stunden bei −10 °C gerührt. Zur Isolierung des Produktes wurden 100 ml Wasser zugesetzt. Umkristallisation des Produktes aus 13 ml Acetonitril lieferte 0,42 g (21%) α-(1-Methyl-2-nitroimidazol-5-yl)-1-pyrrolidin-p-acetanisidid, Smp. 141–143 °C.

Beispiel 8

Zu einer Suspension von 0,14 g 1-Methyl-2-nitroimidazol-5-methanol, 0,22 g 4′-Hydroxy-1-pyrrolidin-acetanilid und 0,26 g Triphenylphosphan in 3 ml absolutem THF wurden unter Kühlung mit Eis und starkem Rühren 0,17 g Azodicarbonsäure-diethylester in 1,5 ml THF zugetropft. Das Gemisch wurde während 3 Stunden bei Raumtemperatur gerührt und die entstandene klare Lösung unter vermindertem Druck eingedampft. Das Rohprodukt wurde durch Chromatographie an einer Kieselgelsäule gereinigt. Nebenprodukte wurden zunächst mit Essigester eluiert, während das Hauptprodukt durch Elution mit Dichlormethan/Methanol (9:1, v/v) erhalten wurde. Abdampfen des Lösungsmittelgemischs und Kristallisation aus 1 ml Acetonitril lieferte 0,115 g (36%) α-(1-Methyl-2-nitroimidazol-5-yl) -1-pyrrolidin-p-acetanisidid, Smp. 140–142 °C.

Beispiel 9

Tablette enthaltend

| | |
|---|---|
| 2-(Dimethylamino) -α- (1-methyl-2-nitro-imidazol-5-yl)- p-acetanisidid-hydrochlorid | 100 mg |
| Lactose | 192 mg |
| Maisstärke | 80 mg |
| Hydrolysierte Maisstärke | 20 mg |
| Calciumstearat | 8 mg |
| | 400 mg |

Beispiel 10

Tablette enthaltend

| | |
|---|---|
| α-(1-Methyl-2-nitroimidazol-5-yl)-1-pyrrolidin-p-acetanisididhydrochlorid | 50 mg |
| Lactose | 194 mg |
| Vorgelatinierte Maisstärke | 150 mg |
| Calciumstearat | 6 mg |
| | 400 mg |

Beispiel 11

Injektionslösung enthaltend pro ml

| | |
|---|---|
| 2-(Dimethylamino) -α-(1-methyl -2-nitro-imidazol-5-yl) -p-acetanisidid | 5.1 mg |
| Propylenglykol | 0,4 ml |
| Benzylalkohol (frei von Benzaldehyd) | 0,015 ml |
| Ethanol (wasserfrei) | 0,10 ml |
| Natriumbenzoat | 48,8 mg |
| Benzoesäure | 1,2 mg |
| Wasser (für Injektions zwecke) q.s. | 1,0 ml |

**Patentansprüche für die Vertragsstaaten BE, CH, DE, FR, IT, LI, NL, SE**

1. 2-Nitroimidazole der allgemeinen Formel I

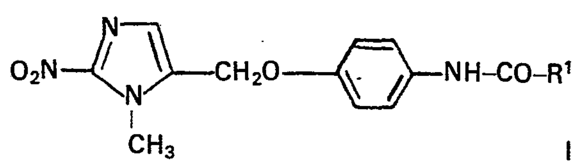

I

worin $R^1$ 1-Methyl-2-pyrrolidinyl oder einen Rest $-CH_2-NR^2R^3$ bedeutet, in dem $R^2$ und $R^3$ jeweils $C_{1-4}$-Alkyl darstellen oder $-NR^2R^3$ der Rest eines fünf- oder sechsgliedrigen gesättigten heterocyclischen Ringsystems mit ggf. einem weiteren Stickstoff- oder Sauerstoffatom im Ring ist und deren physiologisch verträgliche Säureadditionssalze.

2. 2-Nitroimidazole der allgemeinen Formel I gemäss Anspruch 1.

3. 1-Methyl-α- (1-methyl-2-nitroimidazol-5-yl) -pyrrolidin-carboxy-p-anisidid.

4. 2-(Dimethylamino) -α(1-methyl-2-nitroimidazol-5-yl) -p-acetanisidid.

5. 2-(Dimethylamino) -α- (1-methyl-2-nitroimidazol-5-yl) -p-acetanisidid-hydrochlorid.

6. 2-(Diethylamino) -α- (1-methyl-2-nitroimidazol-5-yl) -p-acetanisidid.

7. α- (1-Methyl-2-nitroimidazol-5-yl) -1-pyrrolidin-p-acetanisidid.

8. α- (1-Methyl-2-nitroimidazol-5-yl) -1-pyrrolidin- p-acetanisidid-hydrochlorid.

9. α- (1-Methyl-2-nitroimidazol-5-yl) -1-piperidin-p-acetanisidid.

10. α- (1-Methyl-2-nitroimidazol-5-yl) -4-morpholin-p-acetanisidid.

11. 2-Nitroimidazole gemäss einem der Ansprüche 1–10 als Heilmittel.

12. 2-Nitroimidazole gemäss einem der Ansprüche 1–10 als Heilmittel in der Veterinärmedizin.

13. Verfahren zur Herstellung von Verbindungen gemäss Anspruch 1, dadurch gekennzeichnet, dass man

(a) eine Verbindung der Formel II

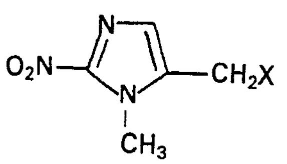

II

in der X Hydroxy, Chlor, Brom oder Jod darstellt, mit einer Verbindung der Formel III

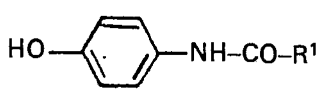

III

umsetzt oder

(b) 2-Halo-α- (1-methyl-2-nitroimidazol-5-yl) -p-acetanisidid mit einer Verbindung $HNR^2R^3$ umsetzt oder

(c) α- (1-Methyl-2-nitroimidazol-5-yl) -p-anisidin mit einer Verbindung $R^1$–COOH oder einem reaktionsfähigen Derivat davon umsetzt und gewünschtenfalls eine erhaltene Verbindung I in ein physiologisch verträgliches Säureadditionssalz überführt.

14. Pharmazeutische Präparate auf der Basis einer Verbindung gemäss den Ansprüchen 1–10.

**Patentansprüche für den Vertragsstaat AT**

1. Verfahren zur Herstellung von 2-Nitroimidazolen der allgemeinen Formel I

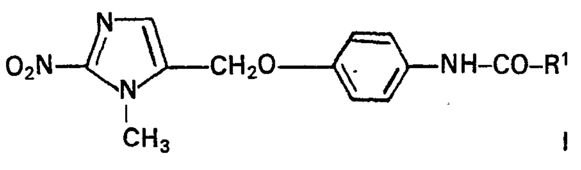

I

worin $R^1$ 1-Methyl-2-pyrrolidinyl oder einen Rest $-CH_2-NR^2R^3$ bedeutet, in dem $R^2$ und $R^3$ jeweils $C_{1-4}$-Alkyl darstellen oder $-NR^2R^3$ der Rest eines fünf- oder sechsgliedrigen gesättigten heterocyclischen Ringsystems mit ggf. einem weiteren Stickstoff- oder Sauerstoffatom im Ring ist, und von deren physiologisch verträglichen Säureadditionssalzen, dadurch gekennzeichnet, dass man

(a) eine Verbindung der Formel II

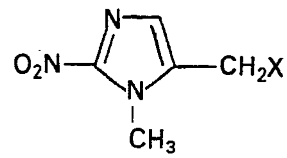

II

in der X Hydroxy, Chlor, Brom oder Jod darstellt, mit einer Verbindung der Formel III

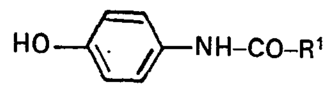

III

umsetzt oder

(b) 2-Halo-α-(1-methyl-2-nitroimidazol-5-yl) -p-acetanisidid mit einer Verbindung $HNR^2R^3$, umsetzt oder

(c) α-(1-Methyl-2-nitroimidazol-5-yl) p-anisidin mit einer Verbindung $R^1$–COOH oder einem reaktionsfähigen Derivat davon umsetzt und gewünschtenfalls eine erhaltene Verbindung I in ein physiologisch verträgliches Säureadditionssalz überführt.

2. Verfahren gemäss Anspruch 1 zur Herstellung einer Verbindung der Formel I.

3. Verfahren gemäss Anspruch 1 zur Herstellung von 1-Methyl-α-(1-methyl-2-nitroimidazol-5-yl) -pyrrolidin-carboxy-p- anisidid.

4. Verfahren gemäss Anspruch 1 zur Herstellung von 2-(Dimethylamino) -α- (1-methyl-2-nitroimidazol-5-yl) -p-acetanisidid.

5. Verfahren gemäss Anspruch 1 zur Herstellung von 2-(Dimethylamino) -α- (1-methyl-2-nitroimidazol-5-yl) -p-acetanisidid-hydrochlorid.

6. Verfahren gemäss Anspruch 1 zur Herstellung von 2-(Diethylamino) -α- (1-methyl-2-nitroimidazol-5-yl) -p-acetanisidid.

7. Verfahren gemäss Anspruch 1 zur Herstellung von α-(1-Methyl-2-nitroimidazol-5-yl) -1-pyrrolidin-p-acetanisidid.

8. Verfahren gemäss Anspruch 1 zur Herstellung von α- (1-Methyl-2-nitroimidazol-5-yl)-1-pyrrolidin-p- acetanisidid-hydrochlorid.

9. Verfahren gemäss Anspruch 1 zur Herstellung von α- (1-Methyl-2-nitroimidazol-5-yl) -1-piperidin-p-acetanisidid.

10. Verfahren gemäss Anspruch 1 zur Herstellung von α-(1-Methyl-2-nitroimidazol-5-yl) -4-morpholin-p-acetanisidid.

**Revendications pour les Etats Contractants: BE, CH, DE, FR, IT, LI, NL, SE**

1. 2-Nitroimidazoles de formule générale I

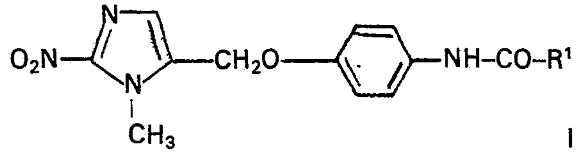

I

dans laquelle $R^1$ est le 1-méthyl-2-pyrrolidinyle ou un reste $-CH_2-NR^2R^3$, dans lequel $R^2$ et $R^3$ représentent chacun un alkyle $C_{1-4}$ ou $-NR^2R^3$ est le reste d'un système circulaire hétérocyclique saturé à cinq ou six maillons avec le cas échéant un autre atome d'azote ou d'oxygène présent dans le cycle, et leurs sels d'addition avec un acide physiologiquement compatibles.

2. 2-Nitroimidazoles de formule générale I selon la revendication 1.

3. 1-Méthyl-α- (1-méthyl-2-nitroimidazole-5-yl) -pyrrolidine-carboxy-p-anisidide.

4. 2-(diméthylamino) -α- (1-méthyl-2-nitroimidazole-5-yl) -p-acétanisidide.

5. Chlorhydrate de 2-(diméthylamino) -α- (1-méthyl-2-nitroimidazole-5-yl) -p-acétanisidide.

6. (2-(diéthylamino) -α- (1-méthyl-2-nitroimidazole-5-yl) -p-acétanisidide.

7. α- (1-méthyl-2-nitroimidazole-5-yl) -1-pyrrolidine-p-acétanisidide.

8. Chlorhydrate d'α- (1-méthyl-2-nitroimidazole-5-yl) -1-pyrrolidine-p-acétanisidide.

9. α- (1-méthyl-2-nitro-imidazole-5-yl) -1-pipéridine-p-acétanisidide.

10. α- (1-méthyl-2-nitro-imidazole-5-yl) -4-morpholine-p-acétanisidide.

11. 2-nitroimidazole selon l'une des revendications 1–10 en tant que médicament.

12. 2-nitroimidazole selon l'une des revendications 1–10 comme médicament en médicine vétérinaire.

13. Procédé de fabrication de composés selon la revendication 1, caractérisé en ce qu'on

(a) fait réagir un composé de formule II

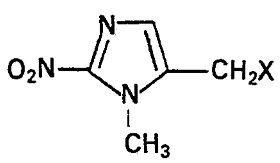

II

dans laquelle X représente un hydroxy, chlore, brome ou iode avec un composé de formule III

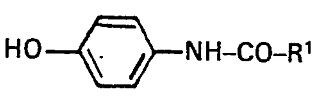

III

ou

(b) fait réagir un 2-halogéno-α- (1-méthyl-2-nitroimidazole-5-yl) -p-acétanisidide avec un composé $HNR^2R^3$ ou

(c) fait réagir le α-(1-méthyl-2-nitroimidazole-5-yl) -p-anisidine avec un composé $R^1-COOH$ ou un de ses dérivés réactifs et si on le souhaite, transforme un composé I obtenue en un sel d'addition avec un acide physiologiquement compatible.

14. Préparations pharmaceutiques à base d'un composé selon les revendications 1–10.

**Revendications pour l'Etat Contractant: AT**

1. Procédé de fabrication de 2-nitroimidazoles de formule générale I

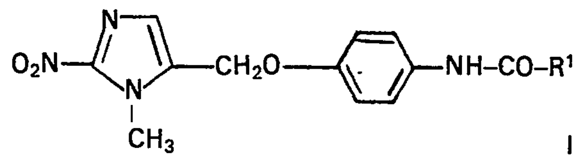

I

dans laquelle $R^1$ est le 1-méthyl-2-pyrrolidinyle ou un reste $-CH_2-NR^2R^3$, dans lequel $R^2$ et $R^3$ représentent chacun un alkyle $C_{1-4}$ ou $-NR^2R^3$ est le reste d'un système circulaire hétérocyclique saturé à cinq ou six maillons avec le cas échéant un autre atome d'azote ou d'oxygène présent dans le cycle, et de leurs sels d'addition avec un acide physiologiquement compatibles, caractérisé en ce qu'on

(a) fait réagir un composé de formule II

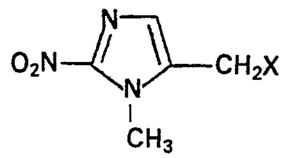

II

dans laquelle X représente un hydroxy, chlore, brome ou iode avec un composé de formule III,

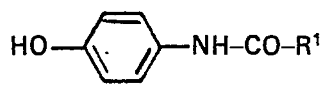

III

ou

(b) fait réagir un 2-halogéno-α- (1-méthyl-2-nitroimidazole-5-yl) -p-acétanisidide avec un composé $HNR^2R^3$, ou

(c) fait réagir le α-(1-méthyl-2-nitroimidazole-5-yl) -p-anisidine avec un composé $R^1-COOH$ ou un de ses dérivés réactifs et si on le souhaite, transforme un composé I obtenu en un sel d'addition avec un acide physiologiquement compatible.

2. Procédé selon la revendication 1 pour fabriquer un composé de formuel I.

3. Procédé selon la revendication 1 pour fabriquer le 1-méthyl-α- (1-méthyl-2-nitroimidazole-5-yl) -pyrrolidine-carboxy-p-anisidide.

4. Procédé selon la revendication 1 pour fabriquer le 2-(diméthylamino) -α- (1-méthyl-2-nitroimidazole-5-yl) -p-acétanisidide.

5. Procédé selon la revendication 1 pour fabriquer le chlorhydrate de 2-(diméthylamino) -α- (1-méthyl-2-nitroimidazole-5-yl) -p-acétanisidide.

6. Procédé selon la revendication 1 pour fabriquer le 2-(diéthylamino) -α- (1-méthyl-2-nitroimidazole-5-yl) -p-acétanisidide.

7. Procédé selon la revendication 1 pour fabriquer le α-(1-méthyl-2-nitroimidazole-5-yl) -1-pyrrolidine-p- acétanisidide.

8. Procédé selon la revendication 1 pour fabriquer le chlorhydrate d'α-(1-méthyl-2-nitroimidazole-5-yl) -1-pyrrolidine-p-acétanisidide.

9. Procédé selon la revendication 1 pour fabriquer le α-(1-méthyl-2-nitroimidazole-5-yl) -1-pipéradine-p-acétanisidide.

10. Procédé selon la revendication 1 pour fabriquer le α-(1-méthyl-2-nitroimidazole-5-yl) 4-morpholine-p-acétanisidide.

**Claims for the Contracting States: BE, CH DE, FR, IT, LI, NL, SE**

1. 2-Nitroimidazoles of general formula I

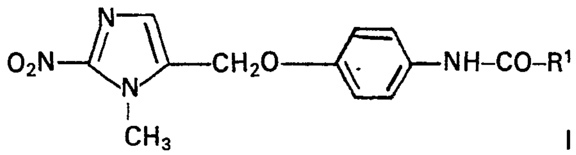

I

wherein $R^1$ signifies 1-methyl-2-pyrrolidinyl or a residue $-CH_2-NR^2R^2$ in which $R^2$ and $R^3$ each represent $C_{1-4}$-alkyl or $-NR^2R^3$ ist the residue of a five – or six-membered saturated heterocyclic ring system with optionally a further nitrogen or oxygen atom in the ring, and their physiologically compatible acid addition salts.

2. 2-Nitroimidazoles of general formula I in accordance with claim 1.

3. 1-Methyl-α- (1-methyl-2-nitroimidazol-5-yl) -pyrrolidine-carboxy-p-anisidide.

4. 2-(Dimethylamino) -α- (1-methyl-2-nitroimidazol-5-yl) p-acetanisidide.

5. 2-(Dimethylamino) -α- (1-methyl-2-nitroimidazol-5-yl) -p-acetanisidide hydrochloride.

6. 2-(Diethylamino) -α- (1-methyl-2-nitroimidazol-5-yl) p-acetanisidide.

7. α-(1-Methyl-2-nitroimidazol-5-yl) -1-pyrrolidine-p- acetanisidide.

8. α-(1-Methyl-2-nitroimidazol-5-yl) 1-pyrrolidine-p- acetanisidide hydrochloride.

9. α- (1-Methyl-2-nitroimidazol-5-yl) -1-piperidine-p- acetanisidide.

10. α-(1-Methyl-2-nitroimidazol-5-yl) -4-morpholine-p-acetanisidide.

11. 2-Nitroimidazoles in accordance with any one of claims 1–10 as medicaments.

12. 2-Nitroimidazoles in accordance with any one of claims 1–10 as medicaments in veterinary medicine.

13. A process for the manufacture of compounds in accordance with claim 1, characterized by

(a) reacting a compound of formula II

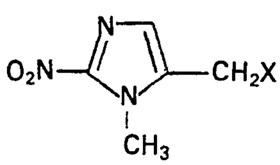

II

in which X represents hydroxy, chlorine, bromine or iodine, with a compound of formula III

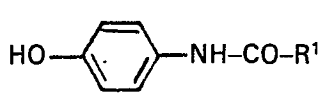

III

or

(b) reacting 2-halo-α- (1-methyl-2-nitroimidazol-5-yl) -p-acetanisidide with a compound $HNR^2R^3$, or

(c) reacting α-(1-methyl-2-nitroimidazol-5-yl) -p-anisidine with a compound $R^1$–COOH or a reactive derivative thereof and, if desired, converting a compound 1 obtained into a physiological compatible acid addition salt.

14. Pharmaceutical preparations based on a compound in accordance with claims 1–10.

**Claims for the Contracting State AT**

1. A process for the manufacture of 2-nitroimidazoles of general formula I

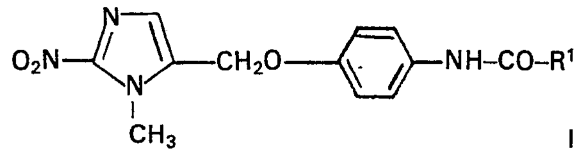

I

wherein $R^1$ signifies 1-methyl-2-pyrrolidinyl or a residue $-CH_2-NR^2R^3$ in which $R^2$ and $R^3$ each represent $C_{1-4}$-alkyl or $-NR^2R^3$ is the residue of a five – or six-membered saturated heterocyclic ring system with optionally a further nitrogen or oxygen atom in the ring, and of their physiologically compatible acid addition salts, characterized by

(a) reacting a compound of formula II

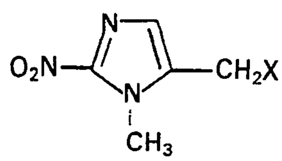

II

in which X represents hydroxy, chlorine, bromine or iodine, with a compound of formula III

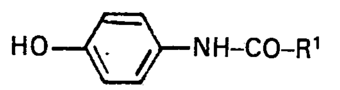

III

or

(b) reacting 2-halo-α- (1-methyl-2-nitroimidazol-5-yl) -p-acetanisidide with a compound $HNR^2R^3$, or

(c) reacting α-(1-methyl-2-nitroimidazol-5-yl) -p-anisidine with a compound $R^1$–COOH or a reactive derivative thereof and, if desired, converting a compound I obtained into a physiological compatible acid addition salt.

2. A process in accordance with claim 1 for the manufacture of a compound of formula I.

3. A process in accordance with claim 1 for the manufacture of 1-methyl-α- (1-methyl-2-nitroimidazol-5-yl) -pyrrolidine-carboxy-p-anisidide.

4. A process in accordance with claim 1 for the manufacture of 2-(dimethylamino) -α- (1-methyl-2-nitroimidazol-5-yl) -p-acetanisidide.

5. A process in accordance with claim 1 for the manufacture of 2-(dimethylamino) -α- (1-methyl-2-nitroimidazol-5-yl) -p-acetanisidide hydrochloride.

6. A process in accordance with claim 1 for the

manufacture of 2-(diethylamino) -α- (1-methyl-2-nitroimidazol-5-yl) -p-acetanisidide.

7. A process in accordance with claim 1 for the manufacture of α-(1-methyl-2-nitroimidazol-5-yl) -1-pyrrolidine-p-acetanisidide.

8. A process in accordance with claim 1 for the manufacture of α-(1-methyl-2-nitroimidazol-5-yl) -1-pyrrolidine-p-acetanisidide hydrochloride.

9. A process in accordance with claim 1 for the manufacture of α-(1-methyl-2-nitroimidazol-5-yl) -1-piperidine-p-acetanisidide.

10. A process in accordance with claim 1 for the manufacture of α-(1-methyl-2-nitroimidazol-5-yl) -4-morpholine-p-acetanisidide.